(19) **Europäisches Patentamt / European Patent Office / Office européen des brevets**

(11) **EP 4 717 178 A1**

(12) **EUROPEAN PATENT APPLICATION**

(43) Date of publication:
01.04.2026 Bulletin 2026/14

(51) International Patent Classification (IPC):
**A61B 6/00** (2024.01)

(21) Application number: 25204523.2

(22) Date of filing: 25.09.2025

(52) Cooperative Patent Classification (CPC):
**A61B 6/482; A61B 6/5217; A61B 6/5282; A61B 6/505**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 26.09.2024 JP 2024167878

(71) Applicant: FUJIFILM Corporation
Tokyo 106-8620 (JP)

(72) Inventor: TAKAHASHI, Tomoyuki
Kanagawa, 258-8538 (JP)

(74) Representative: Dehns Germany Partnerschaft mbB
Theresienstraße 6-8
80333 München (DE)

(54) **RADIATION IMAGE PROCESSING DEVICE, RADIATION IMAGE PROCESSING METHOD, AND RADIATION IMAGE PROCESSING PROGRAM**

(57) A processor acquires two radiation images having a contrast based on a first characteristic corresponding to a first imaging condition, derives a body thickness distribution of a subject based on at least one of the two radiation images, removes a scattered ray component from the two radiation images based on the first characteristic, derives a first characteristic soft part image and a first characteristic bone part image representing a soft part and a bone part of the subject, respectively, from the two radiation images, converts the first characteristic soft part image and the first characteristic bone part image into a second characteristic soft part image and a second characteristic bone part image having a contrast based on a second characteristic corresponding to a second imaging condition, based on the first characteristic, the second characteristic, and the body thickness distribution, and derives a processed target radiation image having the contrast based on the second characteristic by adding the second characteristic soft part image and the second characteristic bone part image.

**FIG. 3**

RADIATION IMAGE PROCESSING DEVICE — 10
- IMAGE ACQUISITION UNIT — 21
- SCATTERED RAY REMOVAL UNIT — 22
- SUBTRACTION UNIT — 23
- CONVERSION UNIT — 24
- DERIVATION UNIT — 25
- DISPLAY CONTROLLER — 26
- CHARACTERISTIC DERIVATION UNIT — 27
- ESTIMATION UNIT — 28
  - ESTIMATION MODEL — 28A

EP 4 717 178 A1

**Description**

BACKGROUND

Technical Field

The present disclosure relates to a radiation image processing device, a radiation image processing method, and a radiation image processing program.

Related Art

In the related art, in a case of making a diagnosis using a radiation image, comparative interpretation using past radiation images of a patient has been performed. Here, in a case in which the two radiation images for the comparative interpretation are acquired by different imaging apparatuses, the contrasts of the two radiation images are different. In addition, in a case in which the imaging condition (a tube voltage of a radiation source, an imaging distance, a tube current, or the like) in a case of imaging differs, the contrasts of the two radiation images will differ. In addition, the contrast of the radiation image is also changed depending on the scattered ray included in the radiation image. In view of such a situation, in order to perform comparative interpretation with high accuracy, a method of matching the contrasts of two radiation images having different imaging conditions and different characteristics of the acquired apparatuses has been proposed (see, for example, JP2023-068496A).

On the other hand, a computer-aided diagnosis (hereinafter, referred to as CAD) system that automatically detects a structure such as an abnormal shadow in a radiation image using a trained estimation model such as artificial intelligence (AI) and performs highlight display of the detected structure has also been proposed.

As described above, in a case where the imaging conditions are different, the contrasts of the acquired radiation images are different. Therefore, in a case of training the AI, it is preferable to use the radiation images acquired under various imaging conditions as teacher data. However, it is difficult to acquire the radiation images under various imaging conditions from the viewpoint of radiation exposure to the human body. Therefore, it is difficult to perform the estimation processing using the estimation model with high accuracy.

SUMMARY OF THE INVENTION

The present disclosure has been made in view of the above circumstances, and an object of the present disclosure is to enable estimation processing using an estimation model to be performed with high accuracy.

A radiation image processing device according to the present disclosure performs estimation processing on a radiation image by using an estimation model constructed by training, and the radiation image processing device includes:

> a processor,
> in which the processor is configured to:

>> acquire two target radiation images having different energy distributions, which are acquired by imaging a subject under a first imaging condition, and a first characteristic corresponding to the first imaging condition;
>> acquire a second characteristic corresponding to a second imaging condition in a case where a teacher radiation image used to construct the estimation model is acquired;
>> derive a body thickness distribution of the subject based on at least one of the two target radiation images;
>> remove a scattered ray component, which is included in radiation transmitted through the subject and is scattered by the subject, from the two target radiation images based on the first characteristic;
>> derive a first characteristic soft part image representing a soft tissue of the subject and a first characteristic bone part image representing a bone tissue of the subject by performing weighted subtraction on the two target radiation images from which the scattered ray component is removed;
>> convert each of the first characteristic soft part image and the first characteristic bone part image into a second characteristic soft part image and a second characteristic bone part image having a contrast based on the second characteristic, based on the first characteristic, the second characteristic, and the body thickness distribution;
>> derive a processed target radiation image having the contrast based on the second characteristic by adding the second characteristic soft part image and the second characteristic bone part image; and
>> input the processed target radiation image to the estimation model to perform the estimation processing.

In the radiation image processing device according to the present disclosure,
the processor may be configured to:

derive a scattered ray component corresponding to the second characteristic based on the second characteristic and the body thickness distribution; and
further derive the processed target radiation image by using the derived scattered ray component.

In the radiation image processing device according to the present disclosure,

the first characteristic may include at least one of an energy of the radiation corresponding to the first imaging condition, a radiation attenuation coefficient corresponding to a body thickness distribution of an object interposed between the subject and a radiation detector that detects the radiation transmitted through the subject in a case where the two target radiation images are acquired, a ratio corresponding to the body thickness distribution of the scattered ray component included in the radiation transmitted through the subject, or a point spread function corresponding to the body thickness distribution, and
the second characteristic may include at least one of an energy of the radiation corresponding to the second imaging condition, a radiation attenuation coefficient corresponding to a teacher body thickness distribution of a teacher subject of an object interposed between the teacher subject and a radiation detector that detects the radiation transmitted through the teacher subject in a case where the teacher radiation image is acquired, a ratio corresponding to the teacher body thickness distribution of the scattered ray component included in the radiation transmitted through the teacher subject, or a point spread function corresponding to the teacher body thickness distribution.

In the radiation image processing device according to the present disclosure, the processor may be configured to display at least one of the two target radiation images and a result of the estimation processing on a display.
A radiation image processing method according to the present disclosure performs estimation processing on a radiation image by using an estimation model constructed by training, and the method includes:

via a computer,
acquiring two target radiation images having different energy distributions, which are acquired by imaging a subject under a first imaging condition, and a first characteristic corresponding to the first imaging condition;
acquiring a second characteristic corresponding to a second imaging condition in a case where a teacher radiation image used to construct the estimation model is acquired;
deriving a body thickness distribution of the subject based on at least one of the two target radiation images;
removing a scattered ray component, which is included in radiation transmitted through the subject and is scattered by the subject, from the two target radiation images based on the first characteristic;
deriving a first characteristic soft part image representing a soft tissue of the subject and a first characteristic bone part image representing a bone tissue of the subject by performing weighted subtraction on the two target radiation images from which the scattered ray component is removed;
converting each of the first characteristic soft part image and the first characteristic bone part image into a second characteristic soft part image and a second characteristic bone part image having a contrast based on the second characteristic, based on the first characteristic, the second characteristic, and the body thickness distribution;
deriving a processed target radiation image having the contrast based on the second characteristic by adding the second characteristic soft part image and the second characteristic bone part image; and
inputting the processed target radiation image to the estimation model to perform the estimation processing.

A radiation image processing program according to the present disclosure causes a computer to execute estimation processing on a radiation image by using an estimation model constructed by training, and the program causes the computer to execute:

a procedure of acquiring two target radiation images having different energy distributions, which are acquired by imaging a subject under a first imaging condition, and a first characteristic corresponding to the first imaging condition;
a procedure of acquiring a second characteristic corresponding to a second imaging condition in a case where a teacher radiation image used to construct the estimation model is acquired;
a procedure of deriving a body thickness distribution of the subject based on at least one of the two target radiation images;
a procedure of removing a scattered ray component, which is included in radiation transmitted through the subject and is scattered by the subject, from the two target radiation images based on the first characteristic;
a procedure of deriving a first characteristic soft part image representing a soft tissue of the subject and a first characteristic bone part image representing a bone tissue of the subject by performing weighted subtraction on the two target radiation images from which the scattered ray component is removed;
a procedure of converting each of the first characteristic soft part image and the first characteristic bone part image into a second characteristic soft part image and a second characteristic bone part image having a contrast based on the second characteristic, based on the first characteristic, the second characteristic, and the body thickness distribution; a procedure of deriving a processed target radiation image having the contrast based on the second characteristic by adding the second characteristic soft part image and the second characteristic bone part image; and

a procedure of inputting the processed target radiation image to the estimation model to perform the estimation processing.

The technology of the present disclosure may be applied to a program product.

According to the present disclosure, it is possible to perform the estimation processing using the estimation model with high accuracy.

BRIEF DESCRIPTION OF THE DRAWINGS

FIG. 1 is a schematic block diagram showing a configuration of a radiography system to which a radiation image processing device according to an embodiment of the present disclosure is applied.
FIG. 2 is a diagram showing a schematic configuration of the radiation image processing device according to the embodiment of the present disclosure.
FIG. 3 is a diagram showing a functional configuration of the radiation image processing device according to the embodiment of the present disclosure.
FIG. 4 is a diagram for describing imaging of a standard object.
FIG. 5 is a diagram showing a spectrum of radiation.
FIG. 6 is a diagram showing radiation attenuation coefficients of a soft tissue, a bone tissue, and aluminum of a human body with respect to radiation energy.
FIG. 7 is a diagram showing a relationship between a thickness of the standard object and the radiation attenuation coefficient.
FIG. 8 is a diagram showing a scattered ray model.
FIG. 9 is a diagram schematically showing processing performed by a conversion unit and a derivation unit.
FIG. 10 is a diagram showing a display screen of an estimation result.
FIG. 11 is a flowchart showing processing performed in the present embodiment.

DETAILED DESCRIPTION

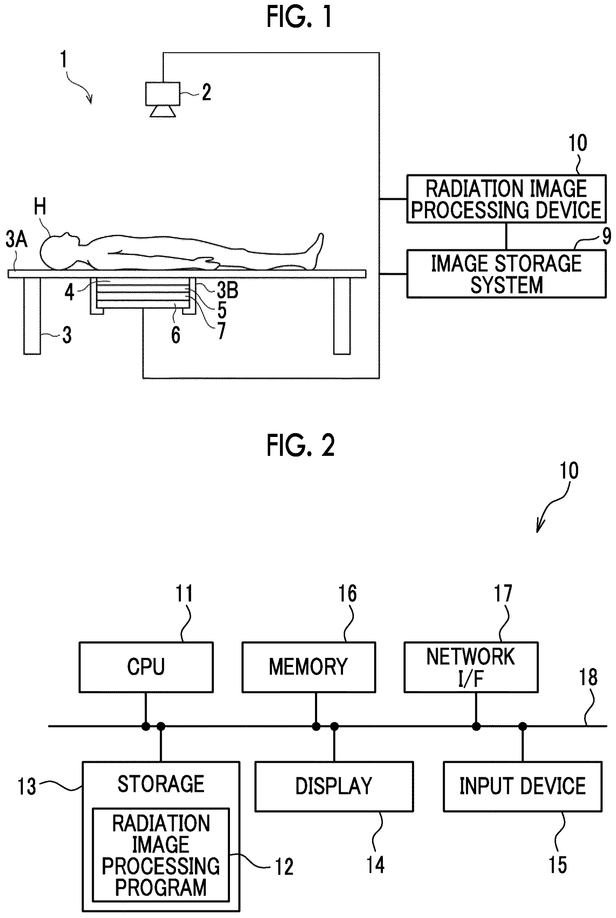

In the following description, an embodiment of the present disclosure will be described with reference to the drawings. FIG. 1 is a schematic block diagram showing a configuration of a radiography system to which a radiation image processing device according to the embodiment of the present disclosure is applied. As shown in FIG. 1, the radiography system according to the present embodiment comprises an imaging apparatus 1, an image storage system 9, and a radiation image processing device 10 according to the present embodiment. The imaging apparatus 1, the image storage system 9, and the radiation image processing device 10 are connected to the image storage system 9 via a network (not shown).

The imaging apparatus 1 is an imaging apparatus that performs energy subtraction by a so-called one-shot method of converting radiation, such as X-rays, emitted from a radiation source 2 and transmitted through a subject H who lies on an imaging table 3 into energy and irradiating a first radiation detector 5 and a second radiation detector 6 with the converted radiation. In a case of imaging, as shown in FIG. 1, a scattered ray removal grid (hereinafter simply referred to as a grid) 4, the first radiation detector 5, a radiation energy conversion filter 7 consisting of a copper plate or the like, and the second radiation detector 6 are disposed in order from a side closest to the radiation source 2, and the radiation source 2 is driven. The first and second radiation detectors 5 and 6 are closely attached to the radiation energy conversion filter 7. Note that the grid 4, the first radiation detector 5, the radiation energy conversion filter 7, and the second radiation detector 6 are attachably and detachably attached below a top plate 3A of the imaging table 3 by an attachment portion 3B.

As a result, in the first radiation detector 5, a first radiation image G1 of the subject H by low-energy radiation also including so-called soft rays is acquired. In addition, in the second radiation detector 6, a second radiation image G2 of the subject H by high-energy radiation from which the soft rays are removed is acquired. The first and second radiation images G1 and G2 are input to the radiation image processing device 10. The first and second radiation images G1 and G2 are examples of two target radiation images according to the present disclosure.

The first and second radiation detectors 5 and 6 can perform recording and reading-out of the radiation image repeatedly. A so-called direct-type radiation detector that directly receives emission of the radiation and generates an electric charge may be used, or a so-called indirect-type radiation detector that converts the radiation into visible light and then converts the visible light into an electric charge signal may be used. In addition, as a method for reading out a radiation image signal, it is desirable to use a so-called thin film transistor (TFT) readout method in which the radiation image signal is read out by turning a TFT switch on and off, or a so-called optical readout method in which the radiation image signal is read out by emission of read out light. However, other methods may also be used without being limited to these methods.

Note that in the imaging apparatus 1, only one radiation detector may be attached to the attachment portion 3B to image the subject H.

The grid 4 is configured by lead that does not transmit the radiation and an interspace material, such as aluminum or fiber that easily transmits the radiation which is disposed alternately with a fine grid density of, for example, about 4.0 lines/mm. By using the grid 4, a scattered ray component of the radiation transmitted through the subject H can be removed, but it cannot be completely removed. Therefore, the first and second radiation images G1 and G2 also include a primary ray component of the radiation transmitted through the subject H as well as the scattered ray component.

The primary ray component is a signal component having a pixel value represented by the radiation that reaches the radiation detector without being scattered by the subject H in the radiation that is transmitted through the subject H. On the other hand, the scattered ray component is a signal component having a pixel value represented by the radiation that reaches the radiation detector by being scattered by the subject H in the radiation that is transmitted through the subject H.

The image storage system 9 is a system that stores image data of the radiation image acquired by the imaging apparatus 1. For example, the image storage system 9 stores a plurality of radiation images for a plurality of patients. The plurality of radiation images include a radiation image used as a teacher radiation image in a case of constructing an estimation model described later. The image storage system 9 extracts an image corresponding to a request from the radiation image processing device 10 from the stored radiation image and transmits the extracted image to a request source device. Specific examples of the image storage system 9 include picture archiving and communication systems (PACS).

Next, the radiation image processing device according to the present embodiment will be described. First, a hardware configuration of the radiation image processing device according to the present embodiment will be described with reference to FIG. 2. As shown in FIG. 2, the radiation image processing device 10 is a computer, such as a workstation, a server computer, and a personal computer, and comprises a central processing unit (CPU) 11, a non-volatile storage 13, and a memory 16 as a transitory storage region. In addition, the radiation image processing device 10 comprises a display 14, such as a liquid crystal display, an input device 15, such as a keyboard and a mouse, and a network interface (I/F) 17 connected to a network (not shown). The CPU 11, the storage 13, the display 14, the input device 15, the memory 16, and the network I/F 17 are connected to a bus 18. It should be noted that the CPU 11 is an example of a processor according to the present disclosure.

The storage 13 is realized by a hard disk drive (HDD), a solid state drive (SSD), a flash memory, and the like. A radiation image processing program 12 installed in the radiation image processing device 10 is stored in the storage 13 as a storage medium. The CPU 11 reads out the radiation image processing program 12 from the storage 13, expands the read out radiation image processing program 12 to the memory 16, and executes the expanded radiation image processing program 12.

It should be noted that the radiation image processing program 12 is stored in a storage device of the server computer connected to the network or in a network storage in a state of being accessible from the outside, and is downloaded and installed in the computer that configures the radiation image processing device 10 in response to the request. Alternatively, the radiation image processing program 12 is distributed in a state of being recorded on a recording medium, such as a digital versatile disc (DVD) or a compact disc read only memory (CD-ROM), and is installed in the computer that configures the radiation image processing device 10 from the recording medium.

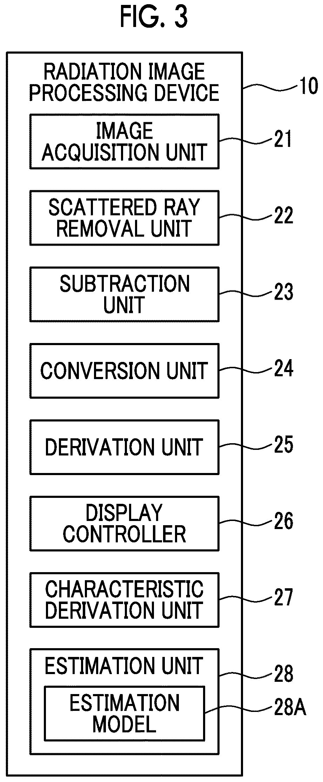

Next, a functional configuration of the radiation image processing device according to the present embodiment will be described. FIG. 3 is a diagram showing the functional configuration of the radiation image processing device according to the present embodiment. As shown in FIG. 3, the radiation image processing device 10 comprises an image acquisition unit 21, a scattered ray removal unit 22, a subtraction unit 23, a conversion unit 24, a derivation unit 25, a display controller 26, a characteristic derivation unit 27, and an estimation unit 28. Moreover, by executing the radiation image processing program 12, the CPU 11 functions as the image acquisition unit 21, the scattered ray removal unit 22, the subtraction unit 23, the conversion unit 24, a derivation unit 25, the display controller 26, the characteristic derivation unit 27, and the estimation unit 28.

The image acquisition unit 21 acquires the first radiation image G1 and the second radiation image G2 of the subject H from the first and second radiation detectors 5 and 6 by causing the imaging apparatus 1 to perform the energy subtraction imaging of the subject H. In a case in which the first radiation image G1 and the second radiation image G2 are acquired, imaging conditions, such as an imaging dose, a radiation quality, a tube voltage (kV), a source image receptor distance (SID) which is a distance between the radiation source 2 and surfaces of the first and second radiation detectors 5 and 6, a source object distance (SOD) which is a distance between the radiation source 2 and a surface of the subject H, and the presence or absence of a scattered ray removal grid are set. The imaging conditions need only be set by input from the input device 15 by an operator.

The SOD and the SID are used to calculate a body thickness distribution as described later. It is preferable that the SOD be acquired by, for example, a time-of-flight (TOF) camera. It is preferable that the SID be acquired by, for example, a potentiometer, an ultrasound range finder, a laser range finder, or the like.

The imaging conditions need only be set by input from the input device 15 by an operator. The set imaging conditions are stored in the storage 13. The first and second radiation images G1 and G2 acquired by the imaging apparatus 1 and the imaging conditions are transmitted to and stored in the image storage system 9.

The scattered ray removal unit 22 removes the scattered ray component from each of the first radiation image G1 and the second radiation image G2 acquired by the image acquisition unit 21. In the following, the removal of the scattered ray component will be described. As a method for removing the scattered ray component, for example, any method, such as a method disclosed in JP2015-043959A, can be used. Hereinafter, scattered ray removal processing in a case in which the method disclosed in JP2015-043959A is used will be described. Note that, in the following description, G1 and G2 will be used as reference numerals for the first and second radiation images from which the scattered ray component is removed.

First, the scattered ray removal unit 22 acquires a virtual model of the subject H having an initial body thickness distribution Ts(x,y). The virtual model is data virtually representing the subject H of which a body thickness in accordance with the initial body thickness distribution Ts(x,y) is associated with a coordinate position of each pixel of the first radiation image G1. Note that the virtual model of the subject H having the initial body thickness distribution Ts(x,y) is stored in the storage 13 in advance, but the virtual model may be acquired from an external server in which the virtual model is stored.

Next, as shown in Expression (1) and Expression (2), the scattered ray removal unit 22 derives an estimated primary ray image Ip(x,y) obtained by estimating a primary ray image obtained by imaging the virtual model and an estimated scattered ray image Is(x,y) obtained by estimating a scattered ray image obtained by imaging the virtual model, based on the virtual model. Further, as shown in Expression (3), the scattered ray removal unit 22 derives an image obtained by combining the estimated primary ray image Ip(x,y) and the estimated scattered ray image Is(x,y) as an estimated image Im(x,y) obtained by estimating the first radiation image G1 obtained by imaging the subject H.

$$Ip(x,y) = Io(x,y) \times \exp(-\mu 1 Soft(T(x,y)) \times T(x,y)) \qquad (1)$$

$$Is(x,y) = Io(x,y) \times STPR1(T(x,y)) * PSF1(T(x,y)) \qquad (2)$$

$$Im(x,y) = Is(x,y) + Ip(x,y) \qquad (3)$$

Here, (x,y) is a coordinate of a pixel position of the first radiation image G1, Io(x,y) is a pixel value of the first radiation image G1 at the pixel position (x,y), Ip(x,y) is the primary ray component at the pixel position (x,y), and Is(x,y) is the scattered ray component at the pixel position (x,y). It should be noted that, in a case of deriving the first estimated image Im(x,y), the initial body thickness distribution Ts(x,y) is used as the body thickness distribution T(x,y) in Expression (1) and Expression (2).

In addition, $\mu 1 Soft(T(x,y))$ in Expression (1) is an attenuation coefficient in accordance with the body thickness distribution (x,y) of the soft tissue of the human body at the pixel position (x,y). The $\mu 1 Soft(T(x,y))$ need only be obtained in advance experimentally or by simulation and stored in the storage 13. In addition, the STPR1(T(x,y)) in Expression (2) is a ratio (scatter-to-primary ratio) of a scattered dose to a primary dose included in the radiation after being transmitted through the subject H having the body thickness distribution T(x,y). The STPR1(T(x,y)) need only also be obtained in advance experimentally or by simulation, and stored in the storage 13.

In addition, the PSF1(T(x,y)) in Expression (2) is a point spread function representing the distribution of the scattered rays spreading from one pixel in accordance with the body thickness distribution T(x,y), and is defined in accordance with the energy characteristic of the radiation. In addition, * is an operator indicating a convolution operation. The PSF1 is also changed due to a distribution of irradiation fields in the imaging apparatus 1, a distribution of the compositions of the subject H, the irradiation dose in a case of imaging, the tube voltage, an imaging distance, the characteristics of the radiation detectors 5 and 6, and the like. Therefore, the PSF1 need only be experimentally obtained in advance for each energy characteristic of the radiation used by the imaging apparatus 1 in accordance with irradiation field information, subject information, the imaging condition, and the like, and stored in the storage 13.

The attenuation coefficient $\mu 1 Soft$, the STPR1, and the PSF1 are examples of first characteristics corresponding to the first imaging condition according to the present disclosure.

Next, the scattered ray removal unit 22 corrects the initial body thickness distribution Ts(x,y) of the virtual model such that a difference between the estimated image Im and the first radiation image G1 is small. The scattered ray removal unit 22 updates the body thickness distribution T(x,y), the scattered ray component Is(x,y), and the primary ray component Ip(x,y) by repeating the derivation of the body thickness distribution T(x,y), the scattered ray component Is(x,y), and the primary ray component Ip(x,y) until a difference between the estimated image Im and the first radiation image G1 satisfies a predetermined termination condition. The scattered ray removal unit 22 subtracts the scattered ray component Is(x,y) derived by Expression (2) from the first radiation image G1 in a case in which the termination condition is satisfied. As a result, the scattered ray component included in the first radiation image G1 is removed. Note that the body thickness distribution T(x,y) derived in a case in which the termination condition is satisfied is used for various calculations described later.

On the other hand, the scattered ray removal unit 22 also performs the scattered ray removal processing on the second radiation image G2 in the same manner as in the first radiation image G1.

In the following, the derivation of the attenuation coefficient $\mu$1Soft and the STPR1 will be described. The attenuation coefficient $\mu$1Soft and the STPR1 are derived by the characteristic derivation unit 27. In a case in which the attenuation coefficient $\mu$1Soft and the STPR1 are derived, the image acquisition unit 21 acquires a standard image K0 by causing the imaging apparatus 1 to image a standard object simulating the human body. In this case, only one radiation detector is used. Note that, in a case in which the standard image K0 is stored in the image storage system 9, the image acquisition unit 21 acquires the standard image K0 from the image storage system 9. In addition, in the following description, the reference numeral "1" is omitted for generalization.

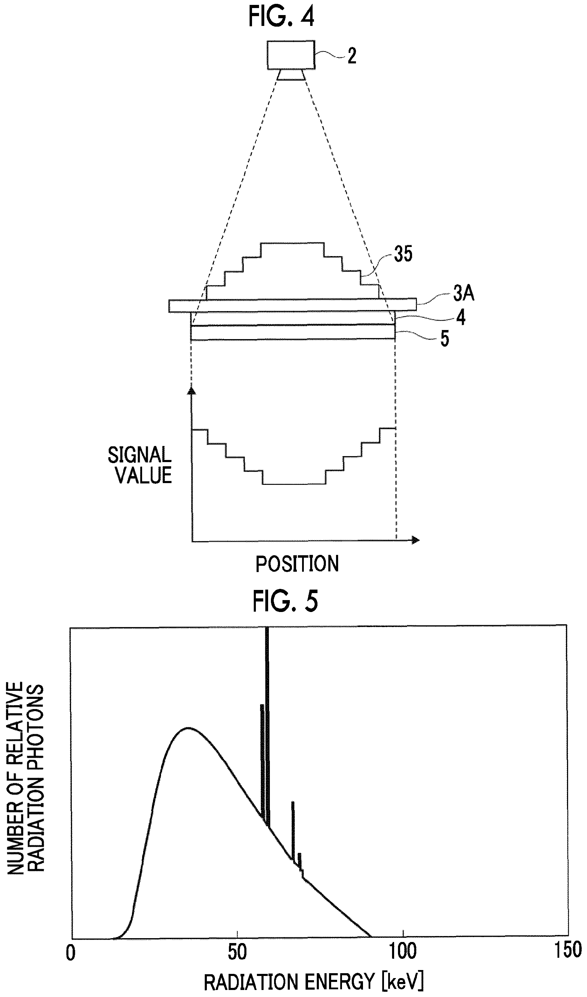

FIG. 4 is a diagram for describing imaging of the standard object. As shown in FIG. 4, a standard object 35 consists of a material having different thickness portions, such as 5 cm, 10 cm, and 20 cm, in stages and having the same radiation transmittance as the soft tissue (fat and muscle) of the human body. Therefore, the standard object 35 simulates a radiation characteristic of the human body. Here, the soft tissue is a mixture of the muscle and the fat in a certain ratio. A mixing ratio of the muscle and the fat differs depending on gender, physique, and the like, but can be defined by an average body fat percentage (25%). Therefore, a material, such as acrylic, which corresponds to the composition mixed at a ratio of 0.75 of the muscle and 0.25 of the fat, is used as the standard object 35.

In a case of acquiring the standard image K0, as shown in FIG. 4, the standard object 35 is placed on the top plate 3A of the imaging table 3, the radiation source 2 is driven to emit the radiation to the radiation detector (here, the first radiation detector 5) via the grid 4, so that the image acquisition unit 21 acquires the standard image K0. The pixel value of each pixel of the standard image K0 includes the primary ray component based on the radiation traveling straight through the standard object 35 and the scattered ray component based on the radiation scattered by the standard object 35.

Note that the standard object 35 is not limited to one object having different thicknesses as shown in FIG. 4. A plurality of standard objects having different thicknesses may be used. In this case, the standard image K0 may be acquired by imaging the plurality of standard objects at once, or the standard images corresponding to each of the standard objects may be acquired by imaging the plurality of standard objects separately.

Also in a case of the acquisition of the standard image K0, the imaging conditions, such as the imaging dose, the tube voltage, the source image receptor distance (SID), which is a distance between the radiation source 2 and the surfaces of the first and second radiation detectors 5 and 6, and the presence or absence of the grid 4, are set.

The characteristic derivation unit 27 acquires the energy characteristic of the radiation in a case of imaging of the standard object 35 in order to derive the attenuation coefficient $\mu$Soft and the STPR. The energy characteristic of the radiation may be acquired from the imaging apparatus 1, or the energy characteristic of the radiation may be stored in the image storage system 9 and acquired from the image storage system 9. Note that a nominal value of the imaging apparatus 1 may be used for the energy characteristic, since there are individual differences in the characteristics of the devices, it is preferable to measure the energy characteristic in advance by using a semiconductor dosimeter.

Here, the energy characteristic is defined by any one of (i) a spectrum of the radiation emitted from the radiation source 2, (ii) the tube voltage [kV] and a total filtration amount [mmAl equivalent], or (iii) the tube voltage [kV] and an aluminum half-value layer [mmAl]. The spectrum of the radiation is obtained by plotting a relationship between the number of relative radiation photons with respect to the radiation energy [keV]. The tube voltage means the maximum value of the generated radiation energy distribution. The total filtration amount is obtained by converting the filtration amount of each constituting component which configures the imaging apparatus 1, such as a radiation generator and a collimator, in the radiation source 2 into a thickness of the aluminum. The influence of the beam hardening in the imaging apparatus 1 is larger and the total amount of high-energy components in the wavelength distribution of the radiation is larger as the total filtration amount is larger. The half-value layer is defined by the thickness of the aluminum necessary to attenuate the dose in half with respect to the generated radiation energy distribution. The high-energy components in the wavelength distribution of the radiation are larger as the aluminum in the half-value layer is thicker.

FIG. 5 is a diagram showing the spectrum of the radiation. In FIG. 5, the spectrum corresponds to the tube voltage of 90 kV and the total filtration amount of 2.5 mmAl. Note that the total filtration amount of 2.5 mmAl corresponds to the half-value layer of 2.96 mmAl.

By using the energy characteristic of the radiation, the characteristic derivation unit 27 derives a relationship between the thickness of the standard object 35 and the radiation attenuation coefficient of the standard object 35, which reflects the influence of the beam hardening of the object present between the standard object 35 and the radiation detector 5.

The characteristic derivation unit 27 first derives the energy spectrum of the radiation from the acquired energy characteristic of the radiation by using a well-known Tucker approximation formula or the like. Note that the acquired energy characteristic is the energy spectrum of the radiation, the acquired energy spectrum need only be used as it is.

Moreover, the characteristic derivation unit 27 derives the radiation attenuation coefficient depending on the thickness of the standard object 35 by simulating the spectrum of the radiation by using a radiation attenuation characteristic of the soft tissue of the human body.

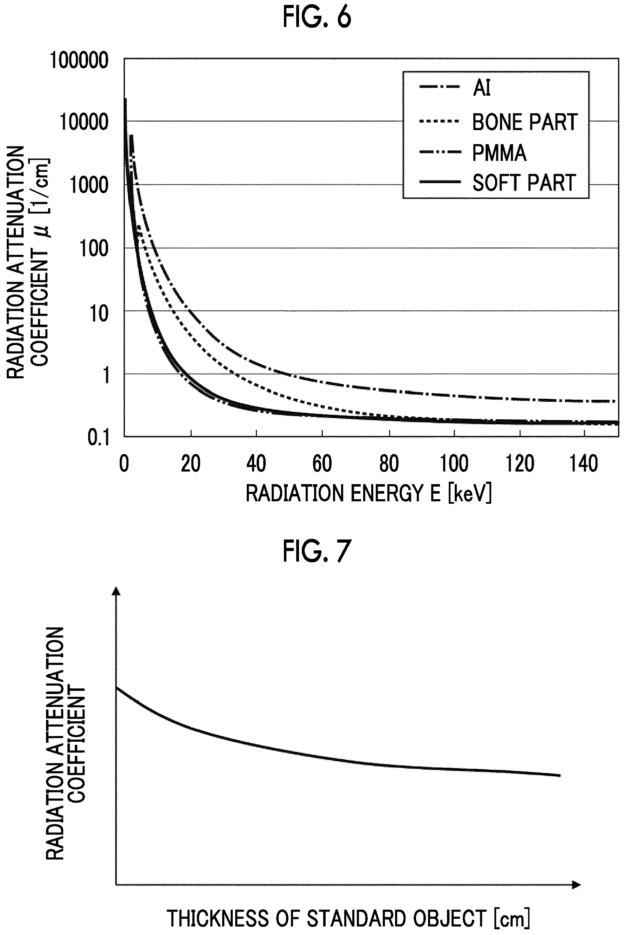

Here, in a case in which the energy spectrum of the radiation emitted from the radiation source 2 is defined as Sin(E) and the thickness of the standard object 35 is defined as t, the radiation dose Xbody(t) after being transmitted through the standard object 35 can be calculated by Expression (4) using a radiation attenuation coefficient μSoft(E) of the soft tissue of the human body. Note that the radiation attenuation coefficients of the soft tissue, the bone tissue, and the aluminum of the human body with respect to the radiation energy are known as shown in FIG. 6, for example. The aluminum is the interspace material for the grid 4. Here, FIG. 6 also shows the radiation attenuation coefficient of the acrylic (polymethyl methacrylate, PMMA), which is the material of the standard object 35. The radiation attenuation coefficient of the acrylic substantially matches the radiation attenuation coefficient of the soft tissue of the human body, as shown in FIG. 6.

$$X_{body}(t) = \int_0^{E} S_{in}(E) \times \exp\{-\mu_{Soft}(E) \times t\} dE \qquad (4)$$

On the other hand, in a case in which the standard object 35 is imaged by the imaging apparatus 1, the top plate 3A and the grid 4 are present between the standard object 35 and the radiation detectors 5 and 6. A material of the top plate 3A is the acrylic and the interspace material of the grid 4 is the aluminum. In a case in which the radiation attenuation coefficient of the acrylic is defined as μPMMA(E), the thickness of the top plate 3A (that is, the thickness of the acrylic) is defined as tPMMA, the radiation attenuation characteristic of the aluminum is defined as μAl(L), and the thickness of grid 4 (that is, the aluminum) is defined as tAl, an X-ray dose Xout(t) after being transmitted through the top plate 3A and the grid 4 is represented by Expression (5).

$$X_{out}(t) = \int_0^{E} S_{in}(E) \times \exp\{-\mu_{Soft}(E) \times t\} \times \exp\{-\mu_{PMMA}(E) \times t_{PMMA}\}$$
$$\times \exp\{-\mu_{Al}(E) \times t_{Al}\} dE \qquad (5)$$

Note that, in a case in which the material of the top plate 3A and the interspace material of the grid 4 are unknown, the X-ray dose Xout(t) after being transmitted through the top plate 3A and the grid 4 cannot be derived by Expression (5). In this case, the energy characteristic (kV, TF0) of the radiation emitted from the radiation source 2 and the energy characteristic (kV, TF1) of the radiation after being transmitted through the top plate 3A and the grid 4 are measured using a dosimeter, and the X-ray dose Xout(t) after being transmitted through the top plate 3A and the grid 4 can be derived by Expression (5-1) using the energy characteristic (kV, TF0) and the energy characteristic (kV, TF1). Note that the energy characteristic in Expression (5-1) represents the total filtration amount (mmAl equivalent) of the radiation emitted by a certain tube voltage [kV].

$$X_{out}(t) = \int_0^{E} S_{in}(E) \times \exp\{-\mu_{Soft}(E) \times t\} \times \exp\{-\mu_{Al}(E) \times (TF1 - TF0)\} dE \qquad (5-1)$$

The radiation attenuation coefficient of the standard object 35 in the imaging system including the top plate 3A and the grid 4 is obtained by representing an attenuation ratio of the radiation after being transmitted through the standard object 35 by an attenuation index as shown in Expression (6) with reference to the radiation dose in a case in which the standard object 35 is not present (that is, in a case in which the thickness of the standard object 35 is 0).

$$\frac{X_{out}(t)}{X_{out}(0)} = \exp\{-\mu_{Soft}(t) \times t\} \qquad (6)$$

By solving Expression (6) with respect to the radiation attenuation coefficient μSoft(t) of the soft tissue as shown in Expression (7), a relationship between a thickness t of the standard object 35 and the radiation attenuation coefficient can be derived.

$$\mu_{Soft}(t) = \frac{\ln\left(\frac{X_{out}(t)}{X_{in}(0)}\right)}{t} \qquad (7$$

The standard object 35 has a plurality of different thicknesses in stages. Therefore, the characteristic derivation unit 27 derives the radiation attenuation coefficient by Expression (7) for each of the plurality of thicknesses of the standard object 35. Moreover, the characteristic derivation unit 27 derives the relationship between the thickness t of the standard object 35 and the radiation attenuation coefficient by performing an interpolation calculation using the radiation attenuation coefficient of the thickness present in the standard object 35 for the radiation attenuation coefficient of the thickness that is not present in the standard object 35. FIG. 7 is a diagram showing a relationship between the thickness t of the standard object 35 and the radiation attenuation coefficient. FIG. 7 shows the relationship between the thickness of the standard object 35 and the radiation attenuation coefficient in a case in which the tube voltage is 90 kV and the total filtration amount is 2.5 mmAl. The characteristic derivation unit 27 derives the relationship between the thickness of the standard object 35 and the radiation attenuation coefficient for each energy characteristic of the radiation, and stores the derived relationship in the storage 13.

The characteristic derivation unit 27 derives the radiation attenuation coefficient corresponding to the thickness of the standard object 35 based on the relationship between the thickness of the standard object 35 and the derived radiation attenuation coefficient. Moreover, the primary ray component included in the standard image K0 is derived based on the radiation attenuation coefficient corresponding to the thickness of the standard object 35.

Here, in a case in which the pixel value of each pixel of the standard image K0 is defined as I0o(x,y), the thickness of the standard object 35 corresponding to each pixel of the standard image K0 is defined as T0(x,y), and the radiation attenuation coefficient derived by Expression (7) with respect to the thickness T0(x,y) of each pixel of the standard image K0 is defined as $\mu$Soft0(x,y), the characteristic derivation unit 27 derives a primary ray component I0p(x,y) included in the pixel value of each pixel of the standard image K0 by Expression (8). Note that since the standard object 35 has the plurality of thicknesses in stages, the characteristic derivation unit 27 derives the primary ray component I0p(x,y) for each thickness present in the standard object 35. Note that the characteristic derivation unit 27 may derive the relationship between the thickness of the standard object 35 and the primary ray component by performing the interpolation calculation using the primary ray component of the thickness that is present in the standard object 35 for the primary ray component corresponding to the thickness that is not present in the standard object 35.

$$I0p(x,y) = I0o(x,y) \times \exp(-\mu Soft0(x,y) \times T0(x,y)) \qquad (8)$$

In addition, the characteristic derivation unit 27 derives the scattered ray component included in the standard object 35 based on the difference between the pixel value of the standard image K0 and the primary ray component. That is, the characteristic derivation unit 27 derives a scattered ray component I0s(x,y) by Expression (9). Note that since the standard object 35 has the plurality of thicknesses in stages, the scattered ray component I0s(x,y) corresponding to the stepwise thickness of the standard object 35 is derived. Note that the characteristic derivation unit 27 need only derive the relationship between the thickness of the standard object 35 and the scattered ray component by performing the interpolation calculation using the scattered ray component of the thickness that is present in the standard object 35 for the scattered ray component corresponding to the thickness that is not present in the standard object 35.

$$I0s(x,y) = I0o(x,y) - I0p(x,y) \qquad (9)$$

The characteristic derivation unit 27 derives a ratio of the scattered ray component I0s(x,y) to the primary ray component I0p(x,y) (that is, I0s(x,y)/I0p(x,y)) for each thickness of the standard object 35 as the STPR. Note that since the thicknesses of the standard object 35 are different in stages, the STPR at the thickness that is not present at the standard object 35 need only be derived by the interpolation calculation using the STPR at the thickness that is present in the standard object 35.

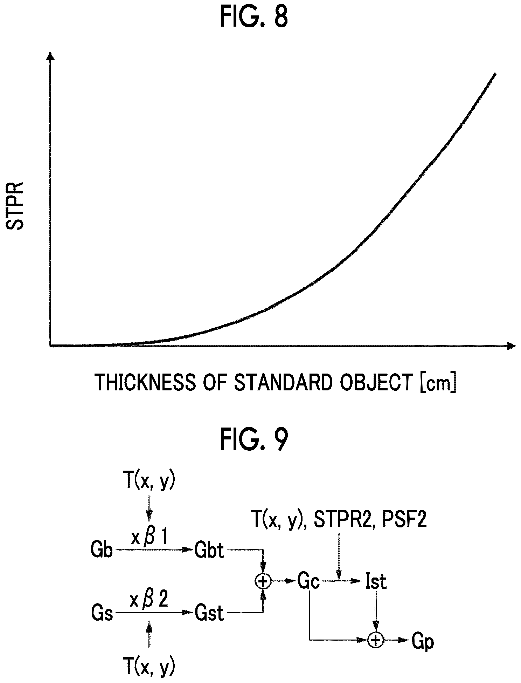

FIG. 8 is a diagram showing a relationship between the thickness of the standard object and the STPR. FIG. 8 shows a relationship between the thickness of the standard object 35 and the STPR in a case in which the tube voltage is 90 kV and the total filtration amount is 2.5 mmAl. The characteristic derivation unit 27 stores the derived scattered ray model in the storage 13. Note that the standard object 35 simulates the radiation characteristic of the human body. Therefore, the relationship between the thickness of the standard object and the STPR shown in FIG. 8 represents a relationship between the thickness of the subject H and the STPR.

Note that the relationship between the thickness of the standard object and STPR need only be derived for each energy characteristic of the radiation that can be emitted by the radiation source 2 of the imaging apparatus 1 and stored in the storage 13.

Here, in the present embodiment, in addition to the radiation attenuation coefficient of the soft tissue, a radiation attenuation coefficient of the bone tissue is also used. Therefore, the characteristic derivation unit 27 also derives the radiation attenuation coefficient of the bone tissue. In the following, the derivation of a radiation attenuation coefficient $\mu 1Bone$ of the bone tissue will be described. Note that the radiation attenuation coefficient $\mu 1Bone$ of the bone tissue is also an example of the first characteristic according to the present disclosure. In addition, in the following description, the reference numeral "1" is omitted for generalization.

Here, a state is assumed in which the soft tissue and the bone tissue overlap on a radiation transmission path, and in a case in which the thickness of the soft tissue is defined as tSoft, the radiation attenuation coefficient can be derived as a function depending on the thickness of the soft tissue. In a case in which the energy spectrum of the radiation emitted from the radiation source 2 is defined as Sin(E) and the thickness of the soft tissue of the subject H is defined as tSoft, a radiation dose Xout1(tSoft) after being transmitted through the subject H in a case in which the bone tissue is not present can be calculated by Expression (10) for each thickness t of the subject H using the radiation attenuation coefficient $\mu$Soft(E) of the soft tissue of the human body. Note that, in Expression (10), as in Expression (5), the radiation attenuation coefficient of the object (that is, the top plate 3A and the grid 4) that is present between the subject H and the radiation detectors 5 and 6 is taken into consideration.

$$X_{out1}(t) = \int_0 S_{in}(E) \times \exp\{-\mu_{Soft}(E) \times t_{Soft}\} \times \exp\{-\mu_{PMMA}(E) \times t_{PMMA}\}$$
$$\times \exp\{-\mu_{Al}(E) \times t_{Al}\}dE \qquad (10)$$

A radiation dose Xout2(t) in a case in which the bone tissue is present is derived by Expression (11) further using a radiation attenuation coefficient $\mu$Bone(E) of the bone tissue.

$$X_{out2}(t) = \int_0 S_{in}(E) \times \exp\{-\mu_{Soft}(E) \times t_{Soft} - \mu_{Bone}(E) \times t_{Bone}\} \times \exp\{-\mu_{PMMA}(E) \times t_{PMMA}\}$$
$$\times \exp\{-\mu_{Al}(E) \times t_{Al}\}dE \qquad (11)$$

The radiation attenuation coefficient of the bone tissue is obtained by representing an attenuation ratio of the radiation dose due to the bone tissue by the attenuation index with reference to the radiation dose in a case in which the bone tissue is not present, as shown in Expression (12).

$$\frac{X_{out2}(t)}{X_{out1}(t)} = \exp\{-\mu_{Bone}(t) \times t_{Bone}\} \qquad (12)$$

By solving Expression (12) for $\mu$Bone(t) as shown in Expression (13), the relationship between the thickness t of the subject H and the radiation attenuation coefficient of the bone tissue can be derived. Note that tBone is the thickness of the bone tissue.

$$\mu_{Bone}(t) = -\frac{\ln\left(\frac{X_{out2}(t)}{X_{out1}(t)}\right)}{t_{Bone}} \qquad (13)$$

Note that the PSF is, as described above, also changed due to a distribution of irradiation fields in the imaging apparatus 1, a distribution of the compositions of the subject H, the irradiation dose in a case of imaging, the tube voltage, an imaging distance, the characteristics of the radiation detectors 5 and 6, and the like. Therefore, the characteristic derivation unit 27 need only experimentally obtain PSF in advance for each energy characteristic of the radiation used by the imaging apparatus 1 in accordance with irradiation field information, subject information, the imaging conditions, and the like, and store the obtained PSF in the storage 13.

Next, the estimation unit 28 will be described. The estimation unit 28 performs, for example, the estimation processing of estimating a region of a specific organ included in the radiation image and a region of a lesion. Therefore, the estimation unit 28 has an estimation model 28A constructed by performing machine learning on a neural network. The estimation model 28A is trained by using a plurality of teacher radiation images and masks of regions, such as lesions, in the teacher radiation images as correct answer data.

In the present embodiment, the teacher radiation image is acquired by imaging the subject under the second imaging condition different from the first imaging condition in a case where the radiation image to be processed is acquired.

Therefore, the estimation model 28A is constructed by performing training using the teacher radiation image having the second characteristic corresponding to the second imaging condition.

In the present embodiment, the characteristic derivation unit 27 derives a radiation attenuation coefficient $\mu$2Soft of the soft tissue, a radiation attenuation coefficient $\mu$2Bone of the bone tissue, the STPR2, and the PSF2 corresponding to the second imaging condition in a case where the teacher radiation image is acquired, for the teacher radiation image, in the same manner as described above. The derived soft tissue radiation attenuation coefficient $\mu$2Soft, bone tissue radiation attenuation coefficient $\mu$2Bone, STPR2, and PSF2 need only be stored in the storage 13. The radiation attenuation coefficient $\mu$2Soft of the soft tissue, the radiation attenuation coefficient $\mu$2Bone of the bone tissue, the STPR2, and the PSF2 for the teacher radiation image are examples of the second characteristic corresponding to the second imaging condition according to the present disclosure.

The subtraction unit 23 performs energy subtraction processing to derive a bone part image Gb in which a bone part of the subject H is extracted and a soft part image Gs in which a soft part is extracted from the first and second radiation images G1 and G2, which are subjected to the scattered ray removal processing. The bone part image Gb and the soft part image Gs are examples of a first bone part image and a first soft part image according to the present disclosure. In the following processing, the first and second radiation images G1 and G2 are radiation images from which the scattered ray component is removed.

In a case in which the bone part image Gb is derived, the subtraction unit 23 performs weighted subtraction between the corresponding pixels with respect to the first and second radiation images G1 and G2 as shown in Expression (14) to generate the bone part image Gb in which the bone part of the subject H included in each of the radiation images G1 and G2 is extracted. In Expression (14), $\alpha$1 is a weighting coefficient, and is set as a value capable of extracting the bone part of the subject H included in each of the radiation images G1 and G2 by Expression (14) based on the radiation attenuation coefficients of the bone tissue and the soft tissue.

$$Gb(x,y) = G1(x,y) - \alpha1 \times G2(x,y) \quad (14)$$

On the other hand, in a case in which the soft part image Gs is derived, the subtraction unit 23 performs weighted subtraction between the corresponding pixels with respect to the first and second radiation images G1 and G2 as shown in Expression (15) to generate the soft part image Gs in which the soft part of the subject H included in each of the radiation images G1 and G2 is extracted. In Expression (15), $\alpha$2 is a weighting coefficient, and is set as a value capable of extracting the soft part of the subject H included in each of the radiation images G1 and G2 by Expression (15) based on the radiation attenuation coefficients of the bone tissue and the soft tissue. The bone part image Gb and the soft part image Gs are examples of a first characteristic bone part image and a first characteristic soft part image according to the present disclosure.

$$Gs(x,y) = G1(x,y) - \alpha2 \times G2(x,y) \quad (15)$$

Next, processing performed by the conversion unit 24 and the derivation unit 25 will be described. FIG. 9 is a diagram schematically showing the processing performed by the conversion unit 24 and the derivation unit 25. First, the processing performed by the conversion unit 24 will be described.

Since the bone part image Gb and the soft part image Gs derived by the subtraction unit 23 are derived from the first and second radiation images G1 and G2 acquired by the imaging apparatus 1 under the first imaging condition, the bone part image Gb and the soft part image Gs have the contrast based on the first characteristic (that is, the radiation energy, the radiation attenuation coefficient, the STPR1, and the PSF1) corresponding to the first imaging condition. The conversion unit 24 converts the bone part image Gb and the soft part image Gs such that the bone part image Gb and the soft part image Gs derived by the subtraction unit 23 have the contrast based on the second characteristic corresponding to the second imaging condition in a case where the teacher radiation image used to construct the estimation model 28A is acquired.

Regarding the bone part image Gb, the conversion unit 24 converts the contrast of the bone part image Gb by Expression (16) to derive a converted bone part image Gbt. In addition, regarding the soft part image Gs, the conversion unit 24 converts the contrast of the soft part image Gs by Expression (17) to derive a converted soft part image Gst. Note that B1 in Expression (16) is derived by $\beta$1 = $\mu$2Soft(T(x,y))/$\mu$1Soft(T(x,y)), and $\beta$2 in Expression (17) is derived by $\beta$2 = $\mu$2Bone(T(x,y))/$\mu$1Bone(T(x,y)). Note that the body thickness distribution T(x,y) derived by the scattered ray removal unit 22 is used. The converted bone part image Gbt and the converted soft part image Gst are examples of a second characteristic bone part image and a second characteristic soft part image according to the present disclosure.

$$Gbt(x,y) = \beta1 \times Gb(x,y) \quad (16)$$

$$Gst(x,y) = \beta 2 \times Gs(x,y) \qquad (17)$$

The derivation unit 25 derives a composite radiation image Gc by adding the converted bone part image Gbt and the converted soft part image Gst derived by the conversion unit 24 between corresponding pixels.

Here, since the bone part image Gb and the soft part image Gs are derived from the first and second radiation images G1 and G2 from which the scattered ray component is removed, the converted bone part image Gbt, the converted soft part image Gst, and the composite radiation image Gc do not include the scattered ray component. Therefore, the composite radiation image Gc may be used as it is for the comparative interpretation between the first and second radiation images G1 and G2 or the bone part image Gb and the soft part image Gs, but the scattered ray component corresponding to the second characteristic is added to the composite radiation image Gc in the present embodiment.

Therefore, the derivation unit 25 derives a scattered ray image Isc representing the scattered ray component corresponding to the second characteristic by Expression (18) using the second characteristic corresponding to the second imaging condition in a case where the teacher radiation image is acquired, that is, STPR2 and PSF2. The scattered ray component corresponding to the second characteristic is a scattered ray component corresponding to the scattered ray component included in the teacher radiation image used in a case where the estimation model 28A is constructed. Note that in Expression (18), Gc(x,y) is the pixel value of each pixel in the composite radiation image Gc. In addition, the body thickness distribution T(x,y) derived by the scattered ray removal unit 22 is used.

$$Isc(x,y) = Gc(x,y) \times STPR2(T(x,y)) * PSF2(T(x,y)) \qquad (18)$$

Moreover, the derivation unit 25 derives a processed target radiation image Gp by adding corresponding pixels of the composite radiation image Gc and the scattered ray image Isc.

The estimation unit 28 derives estimation results for the first and second radiation images G1 and G2 by inputting the processed target radiation image Gp to the estimation model 28A. The estimation result is, for example, a region of a lesion included in the first and second radiation images G1 and G2 or a region of an organ included in the first and second radiation images G1 and G2.

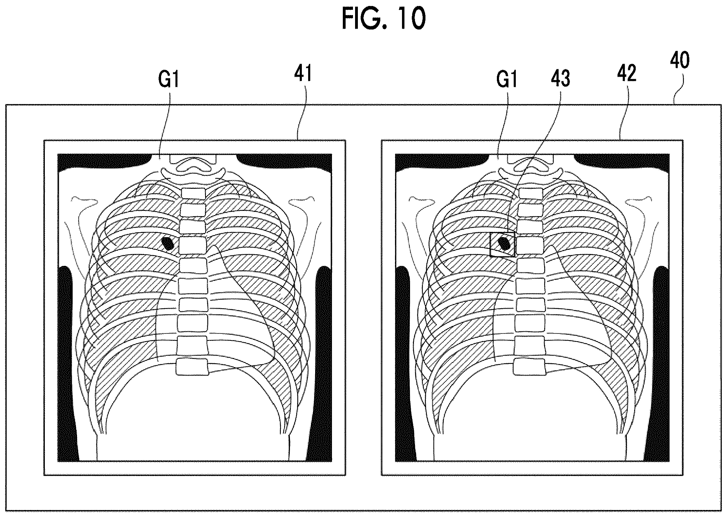

The display controller 26 displays the estimation result on the display 14. FIG. 10 is a diagram showing a display screen of the estimation result. As shown in FIG. 10, the display screen 40 has a first display region 41 in which the first radiation image G1 or the second radiation image G2 is displayed, and a second display region 42 in which the estimation result is displayed. For example, the first radiation image G1 is displayed in the first display region 41. The estimation result estimated by the estimation unit 28 is displayed in the second display region 42. In FIG. 10, a mark 43 is displayed as the estimation result in the region of the lesion in the first radiation image G1.

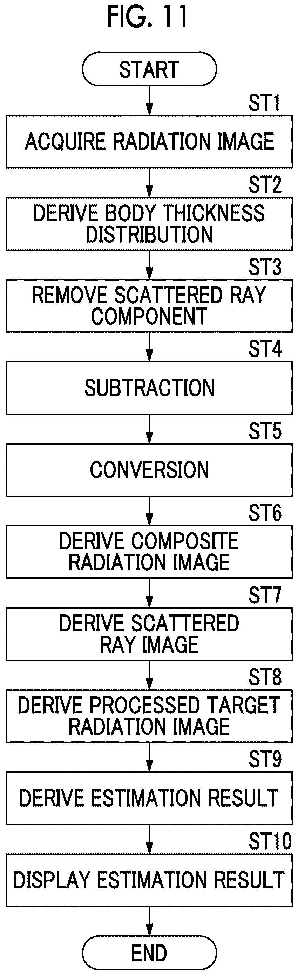

Next, processing performed in the present embodiment will be described. FIG. 11 is a flowchart showing processing performed in the present embodiment. Note that the first and second radiation images G1 and G2 are acquired by the imaging apparatus 1 and stored in the storage 13. In addition, the first characteristic and the second characteristic are acquired by the characteristic derivation unit 27 and stored in the storage 13. In a case in which an instruction to start the processing is input from the input device 15, the image acquisition unit 21 acquires the first and second radiation images G1 and G2 from the storage 13 (radiation image acquisition; step ST1). Next, the scattered ray removal unit 22 derives the body thickness distribution of the subject H from the first and second radiation images G1 and G2 (step ST2), and removes the scattered ray component from each of the first and second radiation images G1 and G2 (step ST3).

Next, the subtraction unit 23 derives the bone part image Gb in which the bone part of the subject H is extracted and the soft part image Gs in which the soft part is extracted from the first and second radiation images G1 and G2 from which the scattered ray component is removed (subtraction; step ST4).

Then, the conversion unit 24 converts the contrasts of the bone part image Gb and the soft part image Gs to derive the converted bone part image Gbt and the converted soft part image Gst (conversion; step ST5). Next, the derivation unit 25 derives the composite radiation image Gc by adding the converted bone part image Gbt and the converted soft part image Gst derived by the conversion unit 24 (step ST6). In addition, the derivation unit 25 also derives the scattered ray image Isc representing the scattered ray component corresponding to the second characteristic (step ST7). Moreover, the derivation unit 25 derives the processed target radiation image Gp by adding the composite radiation image Gc and the scattered ray image Isc (step ST8).

Next, the estimation unit 28 derives the estimation result for the processed target radiation image Gp by using the estimation model 28A (step ST9). Then, the display controller 26 displays the first radiation image G1 or the second radiation image G2 and the estimation result on the display 14 (estimation result display; step ST10), and terminates the processing.

Here, the radiation image acquired by the imaging apparatus 1 includes the scattered ray component corresponding to characteristics, such as the energy of the radiation in a case where the radiation image is acquired, the top plate of the imaging table on which the subject H is placed in the imaging apparatus 1, and the scattered ray removal grid for removing the scattered ray component included in the radiation transmitted through the subject H in a case where the radiation image is acquired. Therefore, the radiation image acquired by the imaging apparatus 1 has the contrast based on the first characteristic corresponding to the first imaging condition in a case where the radiation image is acquired.

On the other hand, the teacher radiation image used to construct the estimation model 28A includes the scattered ray component corresponding to characteristics, such as the energy of the radiation in a case where the teacher radiation image is acquired, the top plate of the imaging table on which the subject H is placed in the imaging apparatus 1 in a case where the teacher radiation image is acquired, and the scattered ray removal grid for removing the scattered ray component included in the radiation transmitted through the subject H in a case where the teacher radiation image is acquired. Therefore, the teacher radiation image has the contrast based on the second characteristic corresponding to the second imaging condition in a case where the teacher radiation image is acquired.

In the present embodiment, the scattered ray component is removed from the first and second radiation images G1 and G2 acquired by the imaging apparatus 1, and the bone part image Gb and the soft part image Gs are derived from the first and second radiation images G1 and G2 from which the scattered ray component is removed. Further, based on the first characteristic, the second characteristic, and the body thickness distribution T(x,y) of the subject H, the bone part image Gb and the soft part image Gs are converted to have the contrast based on the second characteristic corresponding to the second imaging condition in a case where the teacher radiation image is acquired, and the converted bone part image Gbt and the converted soft part image Gst are combined to derive the composite radiation image Gc.

Therefore, in the present embodiment, the radiation image acquired by the imaging apparatus 1 can be converted to have the contrast corresponding to the teacher radiation image used to construct the estimation model 28A. As a result, the contrast of the processed target radiation image Gp to be input to the estimation model 28A can be made to match the contrast of the teacher radiation image. Therefore, according to the present embodiment, the estimation result by the estimation model 28A can be accurately acquired.

In addition, based on the second characteristic and the body thickness distribution, by deriving the scattered ray image representing the scattered ray component corresponding to the second characteristic and deriving the processed target radiation image Gp using the derived scattered ray image, the processed target radiation image Gp can be made to match the contrast of the teacher radiation image including the scattered ray component.

In the above-described embodiment, the estimation model 28A is constructed using the teacher radiation image having the contrast corresponding to the second characteristic corresponding to the second imaging condition. On the other hand, by the processing in the present embodiment, the contrast corresponding to the first characteristic of the radiation image acquired by the imaging apparatus 1 can be converted to have the contrast corresponding to the second characteristic different from the first characteristic. Therefore, the processed target radiation images acquired under various imaging conditions can be derived by using the first and second radiation images G1 and G2 acquired by a single imaging. By constructing the estimation model 28A using the processed target radiation images acquired under various imaging conditions, the estimation model 28A can be constructed such that a highly accurate estimation result can be derived regardless of the imaging conditions. Therefore, it is possible to perform the estimation processing with high accuracy regardless of the imaging conditions of the target radiation image.

Further, in each embodiment described above, the first and second radiation images G1 and G2 are acquired by the one-shot method in a case where the energy subtraction processing is performed, but the present disclosure is not limited thereto. The first and second radiation images G1 and G2 may be acquired by a so-called two-shot method in which imaging is performed twice by using only one radiation detector. In a case of the two-shot method, a position of the subject H included in the first radiation image G1 and the second radiation image G2 may shift due to a body movement of the subject H. Therefore, in the first radiation image G1 and the second radiation image G2, it is preferable to perform the processing according to the present embodiment after registration of the subject is performed.

In addition, in the embodiment described above, bone disease prediction processing is performed by using the radiation image acquired by the system that images the first and second radiation images G1 and G2 of the subject H by using the first and second radiation detectors 5 and 6, it is needless to say that the technology of the present disclosure can be applied to even in a case where the first and second radiation images G1 and G2 are acquired by using an accumulative phosphor sheet instead of the radiation detector. In this case, the first and second radiation images G1 and G2 need only be acquired by stacking two accumulative phosphor sheets, emitting the radiation transmitted through the subject H, accumulating and recording radiation image information of the subject H in each of the accumulative phosphor sheets, and photoelectrically reading the radiation image information from each of the accumulative phosphor sheets. Note that the two-shot method may also be used in a case where the first and second radiation images G1 and G2 are acquired by using the accumulative phosphor sheet.

In addition, the radiation in the embodiment described above is not particularly limited, and $\alpha$-rays or $\gamma$-rays can be used in addition to X-rays.

In the present embodiment, each processing is executed by any computer. In addition, any computer may execute these types of processing by a processor as hardware, a program as software, or a combination thereof. In that case, the processor is configured to execute various types of processing in the present embodiment in cooperation with the program, and can function as each unit or each means in the present embodiment. In addition, the execution order of the processing by the processor is not limited to the order described above and may be appropriately changed. Any computer may be a general-purpose computer, a computer for a specific use, a workstation, or another system capable of executing each processing.

The processor may be configured by one or a plurality of pieces of hardware, and the type of hardware is not limited. For example, the processor may be configured by hardware such as a central processing unit (CPU), a micro processing unit (MPU), a programmable logic device such as a field programmable gate array (FPGA), a dedicated circuit for executing specific processing such as an application specific integrated circuit (ASIC), a graphic processing unit (GPU), or a neural processing unit (NPU). In addition, the types of hardware may be a combination of different types of hardware. In a case where a plurality of pieces of hardware are configured to execute one or a plurality of types of processing of a certain processor, the plurality of pieces of hardware may be present in devices physically separated from each other, or may be present in the same device. In addition, in any of the embodiments, the order of each processing by the processor is not limited to the above order, and may be appropriately changed. The hardware is configured by an electric circuit (circuitry) in which circuit elements such as semiconductor elements are combined.

Furthermore, the program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be divided and stored in the plurality of non-transitory computer-readable media present in devices physically separated from each other. The program code or code segment may represent any combination of a procedure, a function, a subprogram, a routine, a subroutine, a module, a software package, a class, or an instruction, a data structure, or a program statement. The program code or code segment may be connected to another code segment or a hardware circuit by transmitting and receiving information, data, an argument, a parameter, or a content of a memory.

In addition, in the above-described embodiment, the aspect in which the radiation image processing program 12 is stored (installed) in the storage 13 in advance has been described, but the present disclosure is not limited thereto. The radiation image processing program 12 may be provided in a form recorded in a recording medium, such as a compact disc read-only memory (CD-ROM), a digital versatile disc read-only memory (DVD-ROM), and a universal serial bus (USB) memory. Further, the radiation image processing program 12 may also be downloaded from an external device via the network.

The technology of the present disclosure extends to all program products. The program product includes products in all aspects for providing a program. For example, the program product includes a program provided through a network such as the Internet, and a non-transitory computer-readable recording medium such as a CD-ROM, a DVD, and a USB memory in which the program is stored.

The supplementary notes of the present disclosure will be described below.

Supplementary Note 1 A radiation image processing device that performs estimation processing on a radiation image by using an estimation model constructed by training, the radiation image processing device including:

a processor,
in which the processor is configured to:

acquire two target radiation images having different energy distributions, which are acquired by imaging a subject under a first imaging condition, and a first characteristic corresponding to the first imaging condition;
acquire a second characteristic corresponding to a second imaging condition in a case where a teacher radiation image used to construct the estimation model is acquired;
derive a body thickness distribution of the subject based on at least one of the two target radiation images;
remove a scattered ray component, which is included in radiation transmitted through the subject and is scattered by the subject, from the two target radiation images based on the first characteristic;
derive a first characteristic soft part image representing a soft tissue of the subject and a first characteristic bone part image representing a bone tissue of the subject by performing weighted subtraction on the two target radiation images from which the scattered ray component is removed;
convert each of the first characteristic soft part image and the first characteristic bone part image into a second characteristic soft part image and a second characteristic bone part image having a contrast based on the second characteristic, based on the first characteristic, the second characteristic, and the body thickness distribution;
derive a processed target radiation image having the contrast based on the second characteristic by adding the second characteristic soft part image and the second characteristic bone part image; and
input the processed target radiation image to the estimation model to perform the estimation processing.

Supplementary Note 2 The radiation image processing device according to Supplementary Note 1, in which the processor is configured to:

derive a scattered ray component corresponding to the second characteristic based on the second characteristic and the body thickness distribution; and
further derive the processed target radiation image by using the derived scattered ray component.

Supplementary Note 3 The radiation image processing device according to Supplementary Note 1 or 2,

in which the first characteristic includes at least one of an energy of the radiation corresponding to the first imaging condition, a radiation attenuation coefficient corresponding to a body thickness distribution of an object interposed between the subject and a radiation detector that detects the radiation transmitted through the subject in a case where the two target radiation images are acquired, a ratio corresponding to the body thickness distribution of the scattered ray component included in the radiation transmitted through the subject, or a point spread function corresponding to the body thickness distribution, and
the second characteristic includes at least one of an energy of the radiation corresponding to the second imaging condition, a radiation attenuation coefficient corresponding to a teacher body thickness distribution of a teacher subject of an object interposed between the teacher subject and a radiation detector that detects the radiation transmitted through the teacher subject in a case where the teacher radiation image is acquired, a ratio corresponding to the teacher body thickness distribution of the scattered ray component included in the radiation transmitted through the teacher subject, or a point spread function corresponding to the teacher body thickness distribution.

Supplementary Note 4 The radiation image processing device according to any one of Supplementary Notes 1 to 3, in which the processor configured to display at least one of the two target radiation images and a result of the estimation processing on a display.
Supplementary Note 5 A radiation image processing method that performs estimation processing on a radiation image by using an estimation model constructed by training, the method including:

via a computer,
acquiring two target radiation images having different energy distributions, which are acquired by imaging a subject under a first imaging condition, and a first characteristic corresponding to the first imaging condition;
acquiring a second characteristic corresponding to a second imaging condition in a case where a teacher radiation image used to construct the estimation model is acquired;
deriving a body thickness distribution of the subject based on at least one of the two target radiation images;
removing a scattered ray component, which is included in radiation transmitted through the subject and is scattered by the subject, from the two target radiation images based on the first characteristic;
deriving a first characteristic soft part image representing a soft tissue of the subject and a first characteristic bone part image representing a bone tissue of the subject by performing weighted subtraction on the two target radiation images from which the scattered ray component is removed;
converting each of the first characteristic soft part image and the first characteristic bone part image into a second characteristic soft part image and a second characteristic bone part image having a contrast based on the second characteristic, based on the first characteristic, the second characteristic, and the body thickness distribution;
deriving a processed target radiation image having the contrast based on the second characteristic by adding the second characteristic soft part image and the second characteristic bone part image; and
inputting the processed target radiation image to the estimation model to perform the estimation processing.

Supplementary Note 6 A radiation image processing program that causes a computer to execute estimation processing on a radiation image by using an estimation model constructed by training, the program causing the computer to execute:

a procedure of acquiring two target radiation images having different energy distributions, which are acquired by imaging a subject under a first imaging condition, and a first characteristic corresponding to the first imaging condition;
a procedure of acquiring a second characteristic corresponding to a second imaging condition in a case where a teacher radiation image used to construct the estimation model is acquired;
a procedure of deriving a body thickness distribution of the subject based on at least one of the two target radiation images;

a procedure of removing a scattered ray component, which is included in radiation transmitted through the subject and is scattered by the subject, from the two target radiation images based on the first characteristic;

a procedure of deriving a first characteristic soft part image representing a soft tissue of the subject and a first characteristic bone part image representing a bone tissue of the subject by performing weighted subtraction on the two target radiation images from which the scattered ray component is removed;

a procedure of converting each of the first characteristic soft part image and the first characteristic bone part image into a second characteristic soft part image and a second characteristic bone part image having a contrast based on the second characteristic, based on the first characteristic, the second characteristic, and the body thickness distribution;

a procedure of deriving a processed target radiation image having the contrast based on the second characteristic by adding the second characteristic soft part image and the second characteristic bone part image; and

a procedure of inputting the processed target radiation image to the estimation model to perform the estimation processing.

Explanation of References

1: imaging apparatus
2: radiation source
3: imaging table
3A: top plate
3B: attachment portion
4: scattered ray removal grid
5, 6: radiation detector
7: radiation energy conversion filter
9: image storage system
10: radiation image processing device
11: CPU
12: radiation image processing program
13: storage
14: display
15: input device
16: memory
17: network I/F
18: bus
21: image acquisition unit
22: scattered ray removal unit
23: subtraction unit
24: conversion unit
25: derivation unit
26: display controller
27: characteristic derivation unit
28: estimation unit
28A: estimation model
35: standard object
40: display screen
41, 42: image display region
Gb: bone part image
Gp: processed target radiation image
Gs: soft part image

**Claims**

1. A radiation image processing device that performs estimation processing on a radiation image by using an estimation model constructed by training, the radiation image processing device comprising:

a processor,
wherein the processor is configured to:

acquire two target radiation images having different energy distributions, which are acquired by imaging a subject under a first imaging condition, and a first characteristic corresponding to the first imaging condition;
acquire a second characteristic corresponding to a second imaging condition in a case where a teacher radiation image used to construct the estimation model is acquired;
derive a body thickness distribution of the subject based on at least one of the two target radiation images;
remove a scattered ray component, which is included in radiation transmitted through the subject and is scattered by the subject, from the two target radiation images based on the first characteristic;
derive a first characteristic soft part image representing a soft tissue of the subject and a first characteristic bone part image representing a bone tissue of the subject by performing weighted subtraction on the two target radiation images from which the scattered ray component is removed;
convert each of the first characteristic soft part image and the first characteristic bone part image into a second characteristic soft part image and a second characteristic bone part image having a contrast based on the second characteristic, based on the first characteristic, the second characteristic, and the body thickness distribution;
derive a processed target radiation image having the contrast based on the second characteristic by adding the second characteristic soft part image and the second characteristic bone part image; and
input the processed target radiation image to the estimation model to perform the estimation processing.

2. The radiation image processing device according to claim 1,
wherein the processor is configured to:

derive a scattered ray component corresponding to the second characteristic based on the second characteristic and the body thickness distribution; and
further derive the processed target radiation image by using the derived scattered ray component.

3. The radiation image processing device according to claim 1 or 2,

wherein the first characteristic includes at least one of an energy of the radiation corresponding to the first imaging condition, a radiation attenuation coefficient corresponding to a body thickness distribution of an object interposed between the subject and a radiation detector that detects the radiation transmitted through the subject in a case where the two target radiation images are acquired, a ratio corresponding to the body thickness distribution of the scattered ray component included in the radiation transmitted through the subject, or a point spread function corresponding to the body thickness distribution, and
the second characteristic includes at least one of an energy of the radiation corresponding to the second imaging condition, a radiation attenuation coefficient corresponding to a teacher body thickness distribution of a teacher subject of an object interposed between the teacher subject and a radiation detector that detects the radiation transmitted through the teacher subject in a case where the teacher radiation image is acquired, a ratio corresponding to the teacher body thickness distribution of the scattered ray component included in the radiation transmitted through the teacher subject, or a point spread function corresponding to the teacher body thickness distribution.

4. The radiation image processing device according to any one of claims 1 to 3,
wherein the processor is configured to display at least one of the two target radiation images and a result of the estimation processing on a display.

5. A radiation image processing method that performs estimation processing on a radiation image by using an estimation model constructed by training, the method comprising:

via a computer,
acquiring two target radiation images having different energy distributions, which are acquired by imaging a subject under a first imaging condition, and a first characteristic corresponding to the first imaging condition;
acquiring a second characteristic corresponding to a second imaging condition in a case where a teacher radiation image used to construct the estimation model is acquired;
deriving a body thickness distribution of the subject based on at least one of the two target radiation images;
removing a scattered ray component, which is included in radiation transmitted through the subject and is scattered by the subject, from the two target radiation images based on the first characteristic;

deriving a first characteristic soft part image representing a soft tissue of the subject and a first characteristic bone part image representing a bone tissue of the subject by performing weighted subtraction on the two target radiation images from which the scattered ray component is removed;

converting each of the first characteristic soft part image and the first characteristic bone part image into a second characteristic soft part image and a second characteristic bone part image having a contrast based on the second characteristic, based on the first characteristic, the second characteristic, and the body thickness distribution;

deriving a processed target radiation image having the contrast based on the second characteristic by adding the second characteristic soft part image and the second characteristic bone part image; and

inputting the processed target radiation image to the estimation model to perform the estimation processing.

6. A computer-readable storage medium that stores a radiation image processing program that causes a computer to execute estimation processing on a radiation image by using an estimation model constructed by training, the program causing the computer to execute:

a procedure of acquiring two target radiation images having different energy distributions, which are acquired by imaging a subject under a first imaging condition, and a first characteristic corresponding to the first imaging condition;

a procedure of acquiring a second characteristic corresponding to a second imaging condition in a case where a teacher radiation image used to construct the estimation model is acquired;

a procedure of deriving a body thickness distribution of the subject based on at least one of the two target radiation images;

a procedure of removing a scattered ray component, which is included in radiation transmitted through the subject and is scattered by the subject, from the two target radiation images based on the first characteristic;

a procedure of deriving a first characteristic soft part image representing a soft tissue of the subject and a first characteristic bone part image representing a bone tissue of the subject by performing weighted subtraction on the two target radiation images from which the scattered ray component is removed;

a procedure of converting each of the first characteristic soft part image and the first characteristic bone part image into a second characteristic soft part image and a second characteristic bone part image having a contrast based on the second characteristic, based on the first characteristic, the second characteristic, and the body thickness distribution;

a procedure of deriving a processed target radiation image having the contrast based on the second characteristic by adding the second characteristic soft part image and the second characteristic bone part image; and

a procedure of inputting the processed target radiation image to the estimation model to perform the estimation processing.

# FIG. 1

1

2

10

RADIATION IMAGE
PROCESSING DEVICE

9

IMAGE STORAGE
SYSTEM

H

3A

3B
5
7

4

6

3

# FIG. 2

10

11

CPU

16

MEMORY

17

NETWORK
I/F

18

13

STORAGE

RADIATION
IMAGE
PROCESSING
PROGRAM

12

DISPLAY

14

INPUT DEVICE

15

# FIG. 3

RADIATION IMAGE
PROCESSING DEVICE ———10

| IMAGE
ACQUISITION UNIT ——21 |

| SCATTERED RAY
REMOVAL UNIT ——22 |

| SUBTRACTION
UNIT ——23 |

| CONVERSION UNIT ——24 |

| DERIVATION UNIT ——25 |

| DISPLAY
CONTROLLER ——26 |

| CHARACTERISTIC
DERIVATION UNIT ——27 |

| ESTIMATION UNIT ——28
ESTIMATION
MODEL ——28A |

# FIG. 4

SIGNAL VALUE

POSITION

# FIG. 5

NUMBER OF RELATIVE RADIATION PHOTONS

RADIATION ENERGY [keV]

## FIG. 6

## FIG. 7

## FIG. 8

STPR

THICKNESS OF STANDARD OBJECT [cm]

## FIG. 9

$T(x, y)$

$T(x, y)$, STPR2, PSF2

Gb $\xrightarrow{\times \beta 1}$ Gbt

Gs $\xrightarrow{\times \beta 2}$ Gst

$T(x, y)$

$\oplus$ → Gc → Ist

$\oplus$ → Gp

FIG. 10

# FIG. 11

START

ST1

ACQUIRE RADIATION IMAGE

ST2

DERIVE BODY THICKNESS DISTRIBUTION

ST3

REMOVE SCATTERED RAY COMPONENT

ST4

SUBTRACTION

ST5

CONVERSION

ST6

DERIVE COMPOSITE RADIATION IMAGE

ST7

DERIVE SCATTERED RAY IMAGE

ST8

DERIVE PROCESSED TARGET RADIATION IMAGE

ST9

DERIVE ESTIMATION RESULT

ST10

DISPLAY ESTIMATION RESULT

END

**EP 4 717 178 A1**

<table>
<tr><td colspan="4" align="center">**DOCUMENTS CONSIDERED TO BE RELEVANT**</td><td></td></tr>
</table>

EUROPEAN SEARCH REPORT

Application Number

EP 25 20 4523

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/335605 A1 (TAKI TOMOKO [JP]) 20 October 2022 (2022-10-20) * paragraph [0088] - paragraph [0113]; figures * ----- | 1-6 | INV. A61B6/00 |
| A | WO 2019/208037 A1 (SHIMADZU CORP [JP]) 31 October 2019 (2019-10-31) * paragraph [0046] - paragraph [0063] * ----- | 1-6 | |
| A,D | JP 2023 068496 A (FUJIFILM CORP) 17 May 2023 (2023-05-17) * the whole document * ----- | 1-6 | |

TECHNICAL FIELDS SEARCHED (IPC)

A61B
G06N
G06V
G01N

The present search report has been drawn up for all claims

| Place of search | Date of completion of the search | Examiner |
|---|---|---|
| Munich | 10 February 2026 | Strubel, Christine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

26

## ANNEX TO THE EUROPEAN SEARCH REPORT
## ON EUROPEAN PATENT APPLICATION NO.

EP 25 20 4523

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2026

| Patent document cited in search report | | Publication date | Patent family member(s) | | Publication date |
|---|---|---|---|---|---|
| US 2022335605 | A1 | 20-10-2022 | JP | 7686430 B2 | 02-06-2025 |
| | | | JP | 2022163614 A | 26-10-2022 |
| | | | US | 2022335605 A1 | 20-10-2022 |
| WO 2019208037 | A1 | 31-10-2019 | CN | 112165900 A | 01-01-2021 |
| | | | JP | 7092190 B2 | 28-06-2022 |
| | | | JP | WO2019208037 A1 | 01-04-2021 |
| | | | KR | 20200142057 A | 21-12-2020 |
| | | | WO | 2019208037 A1 | 31-10-2019 |
| JP 2023068496 | A | 17-05-2023 | JP | 7737288 B2 | 10-09-2025 |
| | | | JP | 2023068496 A | 17-05-2023 |
| | | | JP | 2025164920 A | 30-10-2025 |
| | | | US | 2023134187 A1 | 04-05-2023 |

EPO FORM P0459

For more details about this annex : see Official Journal of the European Patent Office, No. 12/82

**REFERENCES CITED IN THE DESCRIPTION**

**Patent documents cited in the description**

- JP 2023068496 A
- JP 2015043959 A